# EUROPEAN PATENT APPLICATION

(11) **EP 2 660 304 A1**
(43) Date of publication of application: **06.11.2013**
(21) Application number: 11852892.6
(22) Date of filing: 26.12.2011
(51) Int. Cl.: C10L 3/10, B01J 29/10, C01B 3/34, H01M 8/06

(54) **DESULFURIZATION SYSTEM, HYDROGEN-MANUFACTURING SYSTEM, FUEL-CELL SYSTEM, FUEL-DESULFURIZATION METHOD, AND METHOD FOR MANUFACTURING HYDROGEN**

(30) Priority: 28.12.2010 JP 2010293666
(71) Applicant: JX Nippon Oil & Energy Corporation, Chiyoda-ku Tokyo 100-8162 (JP)
(72) Inventor: MIYAZAWA, Kazunori, Tokyo 100-8162 (JP); KAWABATA, Manabu, Tokyo 100-8162 (JP); SAKODA, Hisao, Tokyo 100-8162 (JP); ISHIZUKI, Kimika, Tokyo 100-8162 (JP)
(74) Representative: Grünecker, Kinkeldey, Stockmair & Schwanhäusser
(86) International application number: PCT/JP2011/080115
(87) International publication number: WO 2012/090957

(57) **Abstract**

A desulfurization system includes: a fuel supply part for supplying a hydrocarbon-based fuel containing water and a sulfur compound to a subsequent stage; and a desulfurization part for desulfurizing the above hydrocarbon-based fuel supplied from the above fuel supply part, wherein, in the above desulfurization part, the above hydrocarbon-based fuel is brought into contact at a temperature of 65 to 105°C with a catalyst prepared by loading silver on an X-type zeolite.

## Description

### Technical Field

The present invention relates to a desulfurization system, a hydrogen-manufacturing system, a fuel-cell system, a fuel-desulfurization method, and a method for manufacturing hydrogen.

### Background Art

As a conventional fuel-cell system, there is known a system comprising a reformer for generating a reformed gas containing hydrogen using a raw fuel, a fuel cell for generating electricity using the reformed gas generated by the reformer, and a desulfurizer for desulfurizing the raw fuel in the upstream of the reformer (for example, refer to Patent Literature 1).

### Citation List

### Patent Literature

Patent Literature 1: Japanese Patent Application Laid-Open No. 2010-27579

### Summary of Invention

### Technical Problem

In the fuel-cell system as described above, if the raw fuel supplied to the desulfurizer contains water, a catalyst for desulfurization received in the desulfurizer may be deactivated to significantly reduce the desulfurization performance. Therefore, in the conventional fuel-cell systems, large-scale dehumidification means or the like is required, or means for reliably preventing the mixing of water in the distribution channel or the like of the raw fuel is required, in order to supply a raw fuel that does not contain water.

Thus, the present invention has an object to provide a desulfurization system which can desulfurize a raw fuel containing water with sufficient desulfurization performance, a hydrogen-manufacturing system for manufacturing hydrogen from the raw fuel desulfurized by this desulfurization system, and a fuel-cell system comprising the above desulfurization system and the above hydrogen-manufacturing system. Further, the present invention has an object to provide a fuel-desulfurization method capable of desulfurizing a raw fuel containing water with sufficient desulfurization performance, and a method for manufacturing hydrogen for manufacturing hydrogen from the raw fuel desulfurized by this desulfurization method.

### Solution to Problem

In one aspect of the present invention, a desulfurization system is characterized by comprising: a fuel supply part for supplying a hydrocarbon-based fuel containing water and a sulfur compound to a subsequent stage; and a desulfurization part for desulfurizing the above hydrocarbon-based fuel supplied from the above fuel supply part, wherein, in the above desulfurization part, the above hydrocarbon-based fuel is brought into contact at a temperature of 65 to 105°C with a catalyst prepared by loading silver on an X-type zeolite.

According to such a desulfurization system, it is possible to desulfurize the hydrocarbon-based fuel with sufficient desulfurization performance even if the hydrocarbon-based fuel supplied from the fuel supply part contains water, by bringing the hydrocarbon-based fuel into contact with a specific catalyst at a specific temperature in the desulfurization part.

In one aspect of the present invention, it is preferable that the above hydrocarbon-based fuel contains a hydrocarbon compound having 4 or less carbon atoms.

In one aspect of the present invention, a hydrogen-manufacturing system comprises: the above desulfurization system; and a hydrogen generation part for generating hydrogen from the above hydrocarbon-based fuel desulfurized in the above desulfurization part.

In such a hydrogen-manufacturing system, since the hydrocarbon-based fuel is desulfurized with sufficient desulfurization performance by the above desulfurization system, the reduction in the hydrogen generation efficiency by a sulfur compound in the above hydrogen generation part is sufficiently suppressed. Therefore, according to the hydrogen-manufacturing system of the present invention, it is possible to efficiently manufacture hydrogen from a hydrocarbon-based fuel containing water and a sulfur compound.

In one aspect of the present invention, a fuel-cell system comprises the above hydrogen-manufacturing system.

According to such a fuel-cell system, since hydrogen is efficiently manufactured by the above hydrogen-manufacturing system, it is possible to achieve good power generation efficiency using a fuel containing water.

One aspect of the present invention also provides a fuel-desulfurization method comprising a step of bringing a hydrocarbon-based fuel containing water and a sulfur compound into contact at a temperature of 65 to 105°C with a catalyst prepared by loading silver on an X-type zeolite.

According to such a fuel-desulfurization method, it is possible to sufficiently suppress the reduction in the desulfurization performance that occurs when using a fuel containing water, by the combination of a specific catalyst and a specific temperature.

In one aspect of the present invention, it is preferable that the above hydrocarbon-based fuel contain a hydrocarbon compound having 4 or less carbon atoms.

One aspect of the present invention further provides a method for manufacturing hydrogen comprising a step of reforming the above hydrocarbon-based fuel desulfurized by the above fuel-desulfurization method to obtain hydrogen.

According to such a method for manufacturing hydrogen, since the above hydrocarbon-based fuel is desulfurized with sufficient desulfurization performance by the above desulfurization method, the reduction in the reforming efficiency by a sulfur compound is sufficiently suppressed, and it is possible to efficiently manufacture hydrogen.

### Advantageous Effects of Invention

According to the present invention, a desulfurization system which can desulfurize a raw fuel containing water with sufficient desulfurization performance, a hydrogen-manufacturing system for manufacturing hydrogen from the raw fuel desulfurized by this desulfurization system, and a fuel-cell system comprising the above desulfurization system and the above hydrogen-manufacturing system are provided. Further, according to the present invention, a fuel-desulfurization method capable of desulfurizing a raw fuel containing water with sufficient desulfurization performance, and a method for manufacturing hydrogen for manufacturing hydrogen from the raw fuel desulfurized by this desulfurization method are provided.

### Brief Description of Drawings

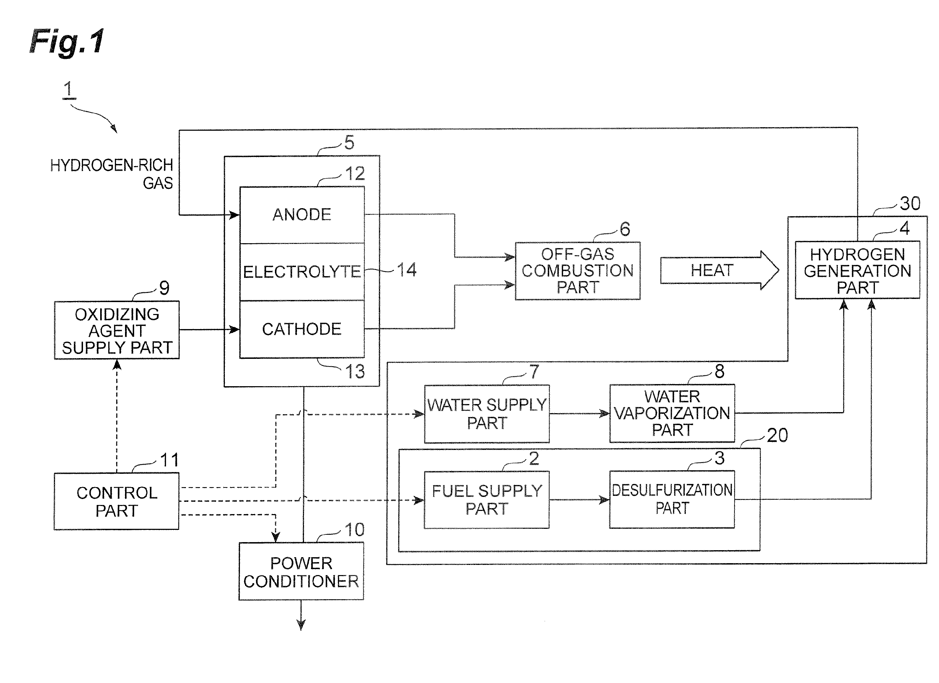

[Figure 1] Figure 1 is a conceptual diagram showing an example of the fuel-cell system according to the embodiments of the present invention.

### Description of Embodiments

Hereinafter, preferred embodiments of the present invention will be described in detail with reference to the drawings.

Figure 1 is a conceptual diagram showing an example of the fuel-cell system according to the embodiments of the present invention. A fuel-cell system 1 comprises a fuel supply part 2, a desulfurization part 3, a hydrogen generation part 4, a cell stack 5, an off-gas combustion part 6, a water supply part 7, a water vaporization part 8, an oxidizing agent supply part 9, a power conditioner 10, and a control part 11, and each part is connected by piping along the flow paths shown in Figure 1.

The fuel supply part 2 constitutes a desulfurization system 20 together with the desulfurization part 3 and supplies a hydrocarbon-based fuel to the desulfurization part 3. Here, as the hydrocarbon-based fuel, it is possible to use a compound containing a carbon atom and a hydrogen atom in the molecule (which may contain other elements such as oxygen) or mixtures thereof. Examples of the hydrocarbon-based fuel include hydrocarbons, alcohols, ethers, and biofuel, and those derived from conventional fossil fuel such as petroleum and coal, those derived from synthetic fuel such as synthesis gas, those derived from biomass, and the like can be suitably used for such hydrocarbon-based fuel.

Examples of the hydrocarbons include hydrocarbon compounds such as methane, ethane, propane, and butane, natural gas, LPG (liquefied petroleum gas), city gas, town gas, gasoline, naphtha, kerosene, and gas oil. Examples of the alcohols include methanol and ethanol. Examples of the ethers include dimethyl ether. Examples of the biofuel include biogas, bioethanol, biodiesel, and bio-jet. In the present embodiments, it is possible to suitably use a gas which is supplied by a pipeline and contains methane as the main ingredient (for example, City gas, Town gas, Natural gas, Biogas, and the like) or LPG.

It is preferable that the hydrocarbon-based fuel contain a hydrocarbon compound having 4 or less carbon atoms. Specific examples of the hydrocarbon compound having 4 or less carbon atoms include saturated aliphatic hydrocarbons such as methane, ethane, propane, and butane, and unsaturated aliphatic hydrocarbons such as ethylene, propylene, and butene. It is preferable that the hydrocarbon-based fuel be a gas containing a hydrocarbon compound having 4 or less carbon atoms, that is, a gas containing one or more of methane, ethane, ethylene, propane, propylene, butane, and butene. Further, as the gas containing a hydrocarbon compound having 4 or less carbon atoms, a gas containing 80% by volume or more of methane is preferred, and a gas containing 85% by volume or more of methane is more preferred.

The hydrocarbon-based fuel supplied from the fuel supply part 2 contains water and a sulfur compound. Here, examples of the water contained in the hydrocarbon-based fuel include water mixed during the manufacture of the hydrocarbon-based fuel, water mixed due to the breakage of a pipeline or the like, and water mixed in the distribution channel until the hydrocarbon-based fuel is supplied to the desulfurization part 3. When the water contained in the hydrocarbon-based fuel is mixed for these reasons, the content of water in the hydrocarbon-based fuel will generally be 0.01 to 2.5% by volume based on the total amount of the hydrocarbon-based fuel. Note that the content of water in the hydrocarbon-based fuel as used in the present specification refers to a value determined from the saturated water vapor pressure calculated from the dew-point temperature measured by a dew-point instrument. According to the desulfurization system according to the present embodiments, it is possible to further suppress the reduction in the desulfurization performance when the content of water is in the above range. The life of a desulfurization catalyst to be described below may be reduced if the content of water exceeds the above range, and therefore, when there is a risk that water exceeding the above range may be contained in the hydrocarbon-based fuel, it is preferable that a simple dehumidification means or the like for controlling the content of water within the above range be provided in the fuel supply part 2. Note that, in order to control the content of the water to less than the above range, a large-scale dehumidification means or the like may be necessary, and productivity will be reduced.

A sulfur compound is generally contained in the hydrocarbon-based fuel. Examples of the sulfur compound include a sulfur compound naturally mixed with hydrocarbons and a compound contained in an odorant for detecting gas leakage. Examples of the sulfur compound naturally mixed with hydrocarbons include hydrogen sulfide (H₂S), carbonyl sulfide (COS), and carbon disulfide (CS₂). As the odorant, an alkyl sulfide or a mercaptan is used by itself or as a mixture, and examples include diethyl sulfide (DES), dimethyl sulfide (DMS), ethyl methyl sulfide (EMS), tetrahydrothiophene (THT), tert-butyl mercaptan (TBM), isopropyl mercaptan, dimethyl disulfide (DMDS), and diethyl disulfide (DEDS). The sulfur compound is generally contained in a concentration of about 0.1 to 10 ppm by mass in terms of sulfur atom based on the total amount of the hydrocarbon-based fuel.

Components other than the above may be contained in the hydrocarbon-based fuel in the range that does not have a bad influence on the characteristics of the fuel-cell system.

The hydrocarbon-based fuel supplied from the fuel supply part 2 is desulfurized in the desulfurization part 3. The sulfur compound contained in the hydrocarbon-based fuel is removed by a desulfurization catalyst in the desulfurization part 3 because it poisons a reforming catalyst in the hydrogen generation part 4 or an electrode catalyst in the cell stack 5. A catalyst prepared by loading silver on an X-type zeolite is used as the desulfurization catalyst.

As the X-type zeolite, it is possible to use, for example, an X-type zeolite in which the ratio of SiO₂/Al₂O₃ is 2 to 3, preferably 2.2 to 3, more preferably 2.3 to 3. When the above ratio is smaller than 2, there is a tendency that the life of the resulting catalyst as a desulfurization catalyst is reduced, and when the above ratio is larger than 3, it may be difficult to load the sufficient amount of silver required for obtaining sufficient desulfurization performance.

The range of the amount of silver loaded is preferably 10 to 30% by mass, more preferably 15 to 25% by mass, based on the total amount of zeolite and silver, from the viewpoint of being further excellent in desulfurization performance. The desulfurization performance may not be sufficient when the amount of silver loaded is less than 10% by mass, and when the amount of silver loaded is more than 30% by mass, the desulfurization performance corresponding to the amount of silver added may not be exhibited. Note that the X-type zeolite can load more silver, for example, as compared with Y-type zeolite.

As a method of loading silver, an ion exchange method is preferably used. The zeolite used in the ion exchange method include various forms such as a sodium type, an ammonium type, and a proton type, and among these, a sodium type is preferably used. On the other hand, silver is generally prepared in a form dissolved in water as a cation. Specific examples thereof include an aqueous solution of silver nitrate or silver perchlorate and an aqueous solution of silver ammine complex ions, and an aqueous silver nitrate solution is most preferably used. The concentration of the aqueous solution containing silver ions is generally in the range of 0.5 to 10% by mass, preferably 1 to 5% by mass, as the concentration of silver.

Although there is no particular limitation on the method of ion exchange, the aforementioned zeolite is generally added to the above solution containing cationic silver and subjected to ion exchange treatment in a temperature range of generally 0 to 90°C, preferably 20 to 70°C, for 1 hour to several hours, preferably with stirring. Subsequently, a solid is separated by filtration or other means, and the resulting zeolite is washed with water or the like and then subjected to drying treatment at a temperature of 50 to 200°C, preferably 80 to 150°C. This ion exchange treatment can be performed repeatedly. Next, as long as it is necessary, calcining treatment may be carried out at 200 to 600°C, preferably 250 to 400°C for about several hours. It is possible to obtain a target silver ion-exchanged zeolite (silver-loading zeolite) by such a method.

The silver-loading zeolite manufactured by the method as described above can be molded for use, by a conventional method such as extrusion molding, tableting molding, rolling granulation, and spray drying, with optional calcining, using alumina, silica, a clay mineral, or the like, or a precursor thereof such as boehmite, as a suitable binder.

In the desulfurization part 3, the hydrocarbon-based fuel is desulfurized by bringing the hydrocarbon-based fuel into contact with the above desulfurization catalyst at a temperature of 65 to 105°C, preferably at a temperature of 70 to 100°C, more preferably at a temperature of 85 to 95°C.

In the desulfurization part 3, it is preferable to set various conditions other than the desulfurization temperature as follows. That is, when using a hydrocarbon-based fuel which is gas at ordinary temperature (for example, 25°C) and normal pressure (for example, a gauge pressure of 0 MPa) such as city gas, it is preferable to select GHSV from the range between 10 and 20000 h⁻¹, preferably between 10 and 7000 h⁻¹. If the GHSV is lower than 10 h⁻¹, the desulfurization performance will be good, but since a large amount of desulfurization catalyst is used, it will be necessary to use an oversized desulfurizer as the desulfurization part 3. Further, the desulfurization performance of the desulfurization part 3 is further improved by setting GHSV to 20000 h⁻¹ or less. Note that it is also possible to use a liquid fuel as the hydrocarbon-based fuel, and in this case, it is preferable to select LHSV between 0.01 and 100 h⁻¹.

The working pressure is selected generally in the range of normal pressure to 1 MPa (gauge pressure, hereinafter the same meaning shall apply), preferably normal pressure to 0.5 MPa, more preferably normal pressure to 0.2 MPa, and the desulfurization can be performed most preferably under atmospheric pressure conditions.

Here, conventionally, when using the desulfurization catalyst as described above, it is common to set the desulfurization temperature to about 30 to 60°C because when the desulfurization temperature is increased, there is a tendency that the desulfurization performance of the desulfurization catalyst may be reduced. However, in such a temperature range, when using a hydrocarbon-based fuel containing water, the desulfurization performance will be significantly reduced.
The cause is considered as follows.

That is, the water contained in the hydrocarbon-based fuel is generally present as vaporized water before being supplied to the desulfurization part 3, but this water may be liquefied and aggregated by capillary action in pores which the desulfurization catalyst has. If water is liquefied and aggregated, it will be impossible for the desulfurization catalyst to adsorb a sulfur compound, and the desulfurization performance will be reduced.

On the other hand, in the present invention, the reduction in the desulfurization performance is suppressed by suppressing the liquefaction and aggregation of water by setting the desulfurization temperature to 65 to 105°C which is higher than before. That is, the present invention has paid attention to two trade-off relations, that is, the reduction in the desulfurization performance with the increase in temperature and the prevention of liquefaction and aggregation of water with the increase in temperature, and has found that the desulfurization performance is improved in a specific temperature region of 65 to 105°C when using the above specific catalyst.

In the present invention, if the desulfurization temperature is lower than 65°C, the liquefaction and aggregation of water cannot sufficiently be suppressed, and the desulfurization performance will be reduced. Further, if the desulfurization temperature is higher than 105°C, the desulfurization performance will be reduced because the reduction in the desulfurization performance with the increase in temperature outweighs the effect of suppression of the liquefaction and aggregation of water.

Note that not all the conventional desulfurization catalysts necessarily have a desulfurization temperature region in which the desulfurization performance is good. Usually, the desulfurization performance is not sufficiently obtained in any temperature region due to the above two factors to reduce the desulfurization performance, or even if a catalyst has a temperature region as described above, the width of the temperature region may be narrow, and temperature control may be difficult.

That is, it can also be said that the present invention uses the above specific desulfurization catalyst because it has a temperature region in which the desulfurization performance is good. Further, in the present invention, since the desulfurization performance is good in a wide temperature region of 65 to 105°C, temperature control is easy, and there are few risks that the desulfurization performance may be reduced by the temperature change due to an external factor or the like.

The hydrocarbon-based fuel from which a sulfur compound has been removed by the desulfurization part 3 is supplied to the hydrogen generation part 4. The hydrogen generation part 4 constitutes a hydrogen-manufacturing system 30 together with the desulfurization system 20. The hydrogen generation part 4 has a reformer for reforming the hydrocarbon-based fuel after desulfurization with a reforming catalyst, and it generates hydrogen-rich gas. The reforming method in the hydrogen generation part 4 is not particularly limited, and it is possible to employ, for example, steam reforming, partial oxidation reforming, self thermal reforming, and other reforming methods. Further, the reforming temperature is generally 200 to 800°C, preferably 300 to 700°C. Note that the hydrogen generation part 4 may have a constitution for adjusting the properties of the hydrogen-rich gas in addition to the reformer for reforming the hydrocarbon-based fuel with a reforming catalyst, according to the properties of the hydrogen-rich gas that the cell stack 5 requires. For example, when the type of the cell stack 5 is a polymer electrolyte fuel cell (PEFC) or a phosphoric acid fuel cell (PAFC), the hydrogen generation part 4 has a constitution for removing carbon monoxide in the hydrogen-rich gas (for example, a shift reaction part, a preferential oxidation reaction part). The hydrogen generation part 4 supplies the hydrogen-rich gas to an anode 12 of the cell stack 5.

Examples of the reforming catalyst include those having a catalyst carrier containing cerium oxide or rare earth element oxide essentially comprising cerium oxide and an active metal loaded on this carrier.

In the reforming catalyst, it is preferable to use Ru or Rh as the active metal. As the amount of Ru or Rh loaded, it is desirable that the atomic ratio of cerium to Ru or Rh (Ce/Ru or Ce/Rh) be 1 to 250, preferably 2 to 100, more preferably 3 to 50. When this atomic ratio is outside the above range, sufficient catalytic activity may not be obtained, which is not preferred. Further, the amount of Ru or Rh loaded is 0.1 to 3.0% by mass, preferably 0.5 to 2.5% by mass, expressed as Ru or Rh metal equivalent, relative to the catalyst weight (the total weight of the catalyst carrier and the active metal).

A method of loading Ru or Rh on a catalyst carrier is not particularly limited and can be easily performed by applying a known method. Examples thereof include an impregnation method, a precipitation method, a coprecipitation method, a kneading method, an ion exchange method, and a pore-filling method, and in particular, an impregnation method is desirable. A starting material of Ru or Rh in the manufacture of the catalyst is different according to the above loading methods and can be suitably selected, but a chloride of Ru or Rh or a nitrate of Ru or Rh is generally used. For example, when applying the impregnation method, it is possible to illustrate a method of preparing a solution of a salt of Ru or Rh (usually aqueous solution) and impregnating the above carrier with the solution, followed by drying and optional calcining. The calcining is generally performed in an air or nitrogen atmosphere or the like, and although the calcining temperature is not particularly limited as long as it is the decomposition temperature or higher of the above salt, it is desirable that the temperature be generally about 200 to 800°C, preferably about 300 to 800°C, more preferably about 500 to 800°C. Generally, in the present invention, a method is preferably employed in which a catalyst is prepared by loading Ru or Rh on a catalyst carrier and then performing reduction treatment in a reducing atmosphere (usually hydrogen atmosphere) at 400 to 1000°C, preferably 500 to 700°C. Note that the above reforming catalyst may have a form in which other noble metal (platinum, iridium, palladium, or the like) is further loaded.

Further, the catalyst carrier of the reforming catalyst is preferably a carrier containing 5 to 40% by mass of cerium oxide or rare earth element oxide essentially comprising cerium oxide and 60 to 95% by mass of aluminum oxide.

Although the cerium oxide is not particularly limited, cerium dioxide (commonly called ceria) is preferred. The preparation method of the cerium oxide is not particularly limited, and it can be prepared by a known method, for example, by calcining in the air or the like, using, for example, cerium nitrate (Ce(NO₃)₃·6H₂O, Ce(NO₃)₄, or the like), cerium chloride (CeCl₃·nH₂O), cerium hydroxide (Ce(OH)₃, Ce(OH)₄·H₂O, or the like), cerium carbonate (Ce₂(CO₃)₃·8H₂O, Ce₂(CO₃)₃·5H₂O, or the like), cerium oxalate, ammonium cerium oxalate (IV), cerium chloride, or the like, as a starting material.

The rare earth element oxide essentially comprising cerium oxide can be prepared from a salt of a mixed rare earth element essentially comprising cerium. In the rare earth element oxide essentially comprising cerium oxide, the content of cerium oxide is generally 50% by mass or more, preferably 60% by mass or more, more preferably 70% by mass or more. Examples of the rare earth element oxides other than cerium oxide include oxides of each element such as scandium, yttrium, lanthanum, praseodymium, neodymium, promethium, samarium, europium, gadolinium, terbium, dysprosium, holmium, erbium, thulium, ytterbium, and lutetium. Among them, the oxides of each element of yttrium, lanthanum, and neodymium are preferred, and particularly, the oxide of lanthanum is preferred. As a matter of course, the crystalline form is not particularly limited and may be any crystalline form.

The aluminum oxide includes alumina and, in addition to that, also includes double oxides of aluminum and other elements such as silicon, copper, iron, and titanium, and representative examples of the double oxides include silica alumina. Alumina is particularly desirable as the aluminum oxide of the present invention, wherein the alumina is not particularly limited, and those having any crystalline form such as α, β, γ, η, θ, κ, and χ can be used, but γ type is particularly preferred. Further, hydrated alumina such as boehmite, bayerite, and gibbsite can also be used. In the case of silica alumina, it is not particularly limited, and those having any crystalline form can be used. As a matter of course, the aluminum oxide used in the present invention can be used without trouble even if it contains a small amount of impurities.

The compositional proportion of the cerium oxide and the rare earth element oxide essentially comprising cerium oxide in the catalyst carrier used in the present invention is 5 to 40% by mass, preferably 10 to 35% by mass. When the proportion of the cerium oxide and the rare earth element oxide essentially comprising cerium oxide is less than 5% by mass, the carbon deposition-suppressing effect, the activity-promoting effect, and the heat resistance improvement effect in the coexistence of oxygen will be insufficient, which is not preferred; and when the proportion of the cerium oxide and the rare earth element oxide essentially comprising cerium oxide is more than 40% by mass, the surface area of the carrier will be reduced, and sufficient catalytic activity may not be obtained, which is not preferred.

The compositional proportion of the aluminum oxide in the catalyst carrier of the reforming catalyst is 60 to 95% by mass, preferably 65 to 90% by mass. When the compositional proportion of the aluminum oxide is less than 60% by mass, the surface area of the carrier will be reduced, and sufficient catalytic activity may not be obtained, which is not preferred; and when the compositional proportion of the aluminum oxide is more than 95% by mass, the carbon deposition-suppressing effect, the activity-promoting effect, and the heat resistance improvement effect in the coexistence of oxygen will be insufficient, which is not preferred.

A method for manufacturing the catalyst carrier of the reforming catalyst is not particularly limited, and it can be easily manufactured by a known method. For example, it can be manufactured by impregnating the aluminum oxide with an aqueous solution of a salt of cerium or a rare earth element essentially comprising cerium, followed by drying and calcining. As the salt used at this time, a water-soluble salt is preferred, and specific examples of the salt include a salt such as nitrate, chloride, sulfate, and acetate; and nitrate or an organic acid salt that is easily thermally decomposed by calcining to form an oxide is particularly preferred. The calcining is generally performed in an air or oxygen atmosphere or the like, and although the calcining temperature is not particularly limited as long as it is the decomposition temperature or higher of the above salt, it is desirable that the temperature be generally about 500 to 1400°C, preferably 700 to 1200°C. Further, as an alternative method of preparing the carrier, it can be prepared also by a coprecipitation method, a gel kneading method, and a sol-gel method.

Although it is possible to obtain the catalyst carrier in this way, it is preferable to subject the catalyst carrier to calcining treatment in an air or oxygen environment before loading Ru or Rh. The calcining temperature at this time is generally 500 to 1400°C, preferably 700 to 1200°C. Further, it is also possible to add a small amount of binder, for example, silica, cement, or the like to the catalyst carrier for the purpose of increasing the mechanical strength of the catalyst carrier. The shape of the catalyst carrier of the reforming catalyst is not particularly limited and can be suitably selected according to the form for using the catalyst. For example, any shape such as pellet shape, granular shape, honeycomb shape, or sponge shape is employed.

Further, in the hydrogen generation part 4, it is preferable to supply water vapor from the water vaporization part 8 in order to reform the hydrocarbon-based fuel. It is preferable that the water vapor be generated by heating and vaporizing the water supplied from the water supply part 7 in the water vaporization part 8. For the heating of the water in the water vaporization part 8, the heat generated within the fuel-cell system 1 may be used, for example, by recovering the heat from the hydrogen generation part 4, the heat from the off-gas combustion part 6, or the heat of exhaust gas. Further, the water may be separately heated using other heat sources such as a heater and a burner. Note that, in Figure 1, only the heat supplied to the hydrogen generation part 4 from the off-gas combustion part 6 is described as an example, but heat is not limited to this heat.

Hydrogen-rich gas is supplied to the fuel-cell system 1 from the hydrogen-manufacturing system 30 through piping (not shown) which connects the hydrogen-manufacturing system 30 and the cell stack 5. Power generation is performed in the cell stack 5 using this hydrogen-rich gas and an oxidizing agent. The type of the cell stack 5 in the fuel-cell system 1 is not particularly limited, and for example, Polymer Electrolyte Fuel Cell (PEFC), Solid Oxide Fuel Cell (SOFC), Phosphoric Acid Fuel Cell (PAFC), Molten Carbonate Fuel Cell (MCFC), and other types can be employed. Depending on the type of the cell stack 5, the reforming method, and the like, the components shown in Figure 1 may be suitably omitted.

The oxidizing agent is supplied from the oxidizing agent supply part 9 through piping which connects the oxidizing agent supply part 9 and the fuel-cell system 1. As the oxidizing agent, for example, air, pure oxygen gas (may contain impurities which are hardly removed by a conventional removal technique), and oxygen enriched air are used.

The cell stack 5 performs power generation using the hydrogen-rich gas from the hydrogen generation part 4 and the oxidizing agent from the oxidizing agent supply part 9. The cell stack 5 comprises the anode 12 to which the hydrogen-rich gas is supplied, a cathode 13 to which the oxidizing agent is supplied, and an electrolyte 14 arranged between the anode 12 and the cathode 13. The cell stack 5 supplies electric power to the outside through a power conditioner 10. The cell stack 5 supplies the hydrogen-rich gas and the oxidizing agent which were not used for generation of electric power to the off-gas combustion part 6, as an off-gas. Note that a combustion part (for example, a combustor for heating the reformer or the like) which the hydrogen generation part 4 has may be shared with the off-gas combustion part 6.

The off-gas combustion part 6 burns the off-gas supplied from the cell stack 5. The heat generated by the off-gas combustion part 6 is supplied to the hydrogen generation part 4, and is used for generating the hydrogen-rich gas in the hydrogen generation part 4. Further, the fuel supply part 2, the water supply part 7, and the oxidizing agent supply part 9 are constituted, for example, by a pump, and are driven by the control signal from the control part 11.

The power conditioner 10 adjusts the electric power from the cell stack 5 in accordance with the power use state in the outside. The power conditioner 10 performs, for example, processing to convert voltage and processing to convert direct current power into alternating current power.

The control part 11 performs control processing of the whole fuel-cell system 1. The control part 11 is constituted by a device comprising, for example, CPU (Central Processing Unit), ROM (Read Only Memory), RAM (Random Access Memory), and an input/output interface. The control part 11 is electrically connected with the fuel supply part 2, the water supply part 7, the oxidizing agent supply part 9, the power conditioner 10, and other sensors and auxiliary machinery that are not shown. The control part 11 acquires various signals generated in the fuel-cell system 1, and it outputs a control signal to each equipment in the fuel-cell system 1.

As mentioned above, according to the desulfurization system, the hydrogen-manufacturing system, and the fuel-cell system of the present invention, it is possible to stably supply a sufficiently desulfurized hydrocarbon-based fuel even if using a hydrocarbon-based fuel containing water as a raw fuel.

Next, the fuel-desulfurization method and the method for manufacturing hydrogen of the present invention will be described. The fuel-desulfurization method of the present invention comprises a step of bringing a hydrocarbon-based fuel containing water and a sulfur compound into contact at a temperature of 65 to 105°C with a catalyst prepared by loading silver on an X-type zeolite.

The hydrocarbon-based fuel containing water and a sulfur compound includes the hydrocarbon-based fuel as mentioned above.

A specific means to bring the hydrocarbon-based fuel into contact with a catalyst prepared by loading silver on an X-type zeolite (desulfurization catalyst) includes the fuel supply part 2 and the desulfurization part 3 as mentioned above. That is, the hydrocarbon-based fuel is supplied to the desulfurization part 3 by the fuel supply part 2, and the supplied hydrocarbon-based fuel is brought into contact with the desulfurization catalyst in the desulfurization part 3.

Generally, the desulfurization conditions are preferably conditions in which the fuel is in a vaporized state. The desulfurization temperature is 65 to 105°C, preferably 70 to 100°C, more preferably 85 to 95°C.

It is preferable to set various conditions other than the desulfurization temperature as follows. That is, when using a hydrocarbon-based fuel which is gas at ordinary temperature (for example, 25°C) and normal pressure (for example, a gauge pressure of 0 MPa) such as city gas, it is preferable to select GHSV between 10 and 20000 h⁻¹, preferably between 10 and 7000 h⁻¹. If GHSV is lower than 10 h⁻¹, the desulfurization performance will be good, but since a large amount of desulfurization catalyst is used, it will be necessary to use an oversized desulfurizer. Further, the desulfurization performance is further improved by setting GHSV to 20000 h⁻¹ or less. Note that it is also possible to use a liquid fuel as the hydrocarbon-based fuel, and in this case, it is preferable to select LHSV between 0.01 and 100 h⁻¹.

The working pressure is selected generally in the range of normal pressure to 1 MPa (gauge pressure, hereinafter the same meaning shall apply), preferably normal pressure to 0.5 MPa, more preferably normal pressure to 0.2 MPa, and the desulfurization can be performed most preferably under atmospheric pressure conditions.

The method for manufacturing hydrogen of the present invention reforms the hydrocarbon-based fuel desulfurized by the above desulfurization method and generates hydrogen (hydrogen-rich gas). The reforming method is not particularly limited as described above, and it is possible to employ, for example, steam reforming, partial oxidation reforming, self thermal reforming, and other reforming methods. The reforming temperature is generally 200 to 800°C, preferably 300 to 700°C.

As described above, in the reforming catalyst, it is preferable to use Ru or Rh as the active metal, and it is preferable that the catalyst carrier be a carrier containing 5 to 40% by mass of cerium oxide or rare earth element oxide essentially comprising cerium oxide and 60 to 95% by mass of aluminum oxide.

Further, in the reforming, since water vapor is optionally required for reforming the fuel, it is preferable that water vapor be supplied to the hydrogen generation part 4 from the water vaporization part 8. It is preferable that the water vapor be generated by heating and vaporizing the water supplied from the water supply part 7 in the water vaporization part 8.

As mentioned above, the suitable embodiments of the present invention have been described, but the present invention is not limited to the above embodiments.

### Examples

Hereinafter, the present invention will be more specifically described with reference to Examples, but the present invention is not limited to Examples.

### <Preparation of Desulfurization Catalyst A>

To 30 g of silver nitrate, was added 600 ml of distilled water to prepare an aqueous silver nitrate solution. Next, the solution was mixed with 50 g of a commercially available NaX-type zeolite powder in which SiO₂/Al₂O₃ (molar ratio) = 2.5 with stirring to perform ion exchange. Subsequently, the resulting powder was washed with distilled water so that a nitric radical might not remain. After washing, the resulting powder was dried overnight in the air at 180°C. With 30 g of the powdered silver-exchanged zeolite after drying, was mixed 5 g of an alumina binder, and the resulting mixture was extruded into a diameter of 1 mm to form a desulfurization catalyst A (shown as "Ag/X" in Table 1). The amount of silver loaded in the desulfurization catalyst A was 24% by mass based on the total amount of zeolite and silver.

### <Preparation of Desulfurization Catalyst B>

A desulfurization catalyst B (shown as "Ag/Y" in Table 2) was prepared in the same manner as in Example 1 except that 50 g of a commercially available NaY-type zeolite powder in which SiO₂/Al₂O₃ (molar ratio) = 5.5 was used instead of the NaX-type zeolite powder. The amount of silver loaded in the desulfurization catalyst B was 24% by mass based on the total amount of zeolite and silver.

### (Reference Example 1)

A fixed-bed flow-type reaction tube was filled with 6 ml of the desulfurization catalyst A, and methane gas (a methane gas containing DMS (dimethyl sulfide) as a sulfur compound in a concentration of 80 ppm by mass in terms of sulfur atom, and in which the content of water (shown as "H₂O concentration" in Table 1) is 0 ppm by mass), was allowed to flow through the catalyst at GHSV = 5000 h⁻¹, at normal pressure and 30°C. Note that the content of the sulfur compound in the methane gas was set to a higher concentration than a conventional concentration for the purpose of the accelerated durability test. The sulfur concentration at the outlet of the reaction tube was measured by SCD (sulfur Chemiluminescence Detector) gas chromatography. After initiation of the experiment, the flow of the gas was stopped when the concentration of the sulfur compound in the outlet gas in terms of sulfur atom based on the total amount of the hydrocarbon-based fuel was 0.05% by volume or more, and the amount of sulfur trapped by the desulfurization catalyst A from the initiation of the experiment to the completion of the experiment was calculated to determine the desulfurization performance.

### (Comparative Example 1)

An experiment was carried out to determine the desulfurization performance in the same manner as in Reference Example 1 except that a methane gas containing DMS in a concentration of 80 ppm by mass in terms of sulfur atom and 1.0% by volume of water, as the methane gas.

### (Comparative Examples 2 and 3)

Experiments were carried out to determine the desulfurization performance in the same manner as in Comparative Example 1, respectively, except that the desulfurization temperature was set to 60°C (Comparative Example 2) or 120°C (Comparative Example 3).

### (Examples 1 to 3)

Experiments were carried out to determine the desulfurization performance in the same manner as in Comparative Example 1, respectively, except that the desulfurization temperature was set to 70°C (Example 1), 90°C (Example 2), or 100°C (Example 3).

### (Reference Example 2)

An experiment was carried out to determine the desulfurization performance in the same manner as in Reference Example 1 except that the desulfurization catalyst B was used instead of the desulfurization catalyst A, and the desulfurization temperature was set to 60°C.

### (Comparative Example 4)

An experiment was carried out to determine the desulfurization performance in the same manner as in Reference Example 2 except that a methane gas containing DMS in a concentration of 80 ppm by mass in terms of sulfur atom and 1.0% by volume of water, as the methane gas.

### (Comparative Examples 5 to 7)

Experiments were carried out to determine the desulfurization performance in the same manner as in Comparative Example 4, respectively, except that the desulfurization temperature was set to 70°C (Comparative Example 5), 80°C (Comparative Example 6), or 90°C (Comparative Example 7).

The desulfurization performance determined in Reference Example 1, Comparative Examples 1 to 3, and Examples 1 to 3, respectively, was evaluated by relative comparison, assuming the performance of Comparative Example 2 as 1. The evaluation results were as shown in Table 1.

**[Table 1]**

| | Catalyst | H₂O concentration (vol%) | Desulfurization temperature (°C) | Desulfurization performance |
|---|---|---|---|---|
| Reference Example 1 | Ag/X | 0 | 30 | 2.0 |
| Comparative Example 1 | Ag/X | 1.0 | 30 | 1.0 |
| Comparative Example 2 | Ag/X | 1.0 | 60 | 1.0 |
| Example 1 | Ag/X | 1.0 | 70 | 1.4 |
| Example 2 | Ag/X | 1.0 | 90 | 1.6 |
| Example 3 | Ag/X | 1.0 | 100 | 1.4 |
| Comparative Example 3 | Ag/X | 1.0 | 120 | 0.6 |

The desulfurization performance determined in Reference Example 2 and Comparative Examples 4 to 7, respectively, was evaluated by relative comparison, assuming the performance of Comparative Example 4 as 1. The evaluation results were as shown in Table 2.

**[Table 2]**

| | Catalyst | H₂O concentration (vol%) | Desulfurization temperature (°C) | Desulfurization performance |
|---|---|---|---|---|
| Reference Example 2 | Ag/Y | 0 | 60 | 2.0 |
| Comparative Example 4 | Ag/Y | 1.0 | 60 | 1.0 |
| Comparative Example 5 | Ag/Y | 1.0 | 70 | 1.2 |
| Comparative Example 6 | Ag/Y | 1.0 | 80 | 1.2 |
| Comparative Example 7 | Ag/Y | 1.0 | 90 | 0.8 |

As shown in Table 1, it was possible to suppress the reduction in the desulfurization performance by setting the desulfurization temperature within the range of 65 to 105°C when desulfurizing the hydrocarbon-based fuel containing water using the desulfurization catalyst A (Ag/X). On the other hand, it was impossible to suppress the reduction in the desulfurization performance when using the desulfurization catalyst B (Ag/Y) as a desulfurization catalyst. Further, as shown in Examples 1 to 3, since it is enough only to hold the desulfurization temperature in a large temperature region of 65 to 105°C in the present invention, a precise temperature control means or the like is not required, which is high in practicality.

### Reference Signs List

1 ... Fuel-cell system, 2 ... Fuel supply part, 3 ... Desulfurization part, 4 ... Hydrogen generation part, 5 ... Cell stack, 20 ... Desulfurization system, 30 ... Hydrogen-manufacturing system.

## Claims

1. A desulfurization system comprising:
a fuel supply part for supplying a hydrocarbon-based fuel containing water and a sulfur compound to a subsequent stage; and
a desulfurization part for desulfurizing the hydrocarbon-based fuel supplied from the fuel supply part,
wherein, in the desulfurization part, the hydrocarbon-based fuel is brought into contact at a temperature of 65 to 105°C with a catalyst prepared by loading silver on an X-type zeolite.

2. The desulfurization system according to claim 1, wherein the hydrocarbon-based fuel contains a hydrocarbon compound having 4 or less carbon atoms.

3. A hydrogen-manufacturing system comprising:
the desulfurization system according to claim 1 or 2; and
a hydrogen generation part for generating hydrogen from the hydrocarbon-based fuel desulfurized in the desulfurization part.

4. A fuel-cell system comprising:
the hydrogen manufacturing-system according to claim 3.

5. A fuel-desulfurization method comprising:
a step of bringing a hydrocarbon-based fuel containing water and a sulfur compound into contact at a temperature of 65 to 105°C with a catalyst prepared by loading silver on an X-type zeolite.

6. The fuel-desulfurization method according to claim 5, wherein the hydrocarbon-based fuel contains a hydrocarbon compound having 4 or less carbon atoms.

7. A method for manufacturing hydrogen comprising:
a step of reforming the hydrocarbon-based fuel desulfurized by the fuel-desulfurization method according to claim 5 or 6 to obtain hydrogen.
